(19) Europäisches Patentamt
European Patent Office
Office européen des brevets

(11) **EP 1 737 515 B1**

(12) **EUROPÄISCHE PATENTSCHRIFT**

(45) Veröffentlichungstag und Bekanntmachung des Hinweises auf die Patenterteilung:
**07.08.2013 Patentblatt 2013/32**

(21) Anmeldenummer: 05730998.1

(22) Anmeldetag: **01.04.2005**

(51) Int Cl.:
***A61M 5/168*** (2006.01)

(86) Internationale Anmeldenummer:
**PCT/EP2005/003446**

(87) Internationale Veröffentlichungsnummer:
**WO 2005/105182 (10.11.2005 Gazette 2005/45)**

(54) **VORRICHTUNG UND VERFAHREN ZUR OKKLUSIONSERKENNUNG BEI INFUSIONSPUMPEN**

DEVICE AND METHOD FOR THE DETECTION OF AN OCCLUSION IN INFUSION PUMPS

DISPOSITIF ET PROCEDE DE DETECTION D'OCCLUSION DANS DES POMPES A PERFUSION

(84) Benannte Vertragsstaaten:
**AT BE BG CH CY CZ DE DK EE ES FI FR GB GR HU IE IS IT LI LT LU MC NL PL PT RO SE SI SK TR**

(30) Priorität: **20.04.2004 DE 102004019053**

(43) Veröffentlichungstag der Anmeldung:
**03.01.2007 Patentblatt 2007/01**

(73) Patentinhaber:
• **F. Hoffmann-La Roche AG**
**4070 Basel (CH)**
Benannte Vertragsstaaten:
**AT BE BG CH CY CZ DK EE ES FI FR GB GR HU IE IS IT LI LT LU MC NL PL PT RO SE SI SK TR**
• **Roche Diagnostics GmbH**
**68305 Mannheim (DE)**
Benannte Vertragsstaaten:
**DE**

(72) Erfinder: **REMDE, Axel**
**CH-3432 Lützelflüh-Goldbach (CH)**

(74) Vertreter: **Rentsch Partner AG**
**Rechtsanwälte und Patentanwälte**
**Fraumünsterstrasse 9**
**Postfach 2441**
**8022 Zürich (CH)**

(56) Entgegenhaltungen:
| | |
|---|---|
| US-A- 4 882 575 | US-A- 5 501 665 |
| US-A- 5 808 203 | US-A- 5 906 589 |
| US-A1- 2003 073 954 | US-B1- 6 416 291 |

**Beschreibung**

Die Erfindung bezieht sich auf eine Vorrichtung und Verfahren zur Okklusionserkennung bei Infusionspumpen. Okklusionen, d. h. Verstopfungen im Fluidweg, sollen bei tragbaren Infusionspumpen schnellstmöglich detektiert werden, um die Zeit, in der fehlerhaft keine Medikamentenausschüttung erfolgt, zu minimieren.

Insbesondere bezieht sich die Erfindung auf das Gebiet miniaturisierter Infusionspumpen, die vom Patienten permanent am Körper getragen werden und eine (quasi) kontinuierliche Medikamentenförderung gewährleisten. Eine besondere Gefahr dieser Systeme liegt in möglichen Verstopfungen (Okklusionen) entweder des Katheters oder der Infusionskanüle. Die hieraus resultierende Unterbrechung der Förderung kann bei längerem Andauern zu einer potentiell lebensgefährlichen Situation für den Patienten führen. Löst sich eine vorübergehende Okklusion vor ihrer Detektion, wird die gesamte angestaute Medikamentenmenge spontan abgegeben. Diese Überdosis kann ebenfalls zu einer Gefahrensituation führen. Bekannt ist diese Problematik z. B. bei der Behandlung von Diabetes Mellitus mittels kontinuierlicher subkutaner Insulininfusien (Continuous Subcutaneous Insulin Infusion, CSII).

Der besondere Nutzen der Erfindung ergibt sich insbesondere bei der kontinuierlichen subkutanen Insulininfusion (CSII) wie folgt:

- Der Patient wird nach dem Auftreten einer Okklusion schneller gewarnt und kann selbstständig geeignete Maßnahmen zu ihrer Behebung (z. B. Wechsel des Katheters) sowie ggf. zur Korrektur eines bereits erhöhten Blutglucoseniveaus ergreifen, bevor eine potentiell gefährliche Situation entsteht. Durch die vorgeschlagene Erfindung wird daher die Sicherheit der Therapie verbessert.
- Da die zur Okklusionserkennung benötigte Zeit mit abnehmender Förderrate steigt, ist sie bei Patienten mit sehr geringem Medikamentenbedarf (z. B. Kindern) besonders kritisch. Nur durch ein sehr schnelles Detektionsverfahren ist eine sichere Therapie in diesen Fällen überhaupt möglich.
- Der grundsätzlich wünschenswerte Einsatz höherer Insulinkonzentrationen wird derzeit u. a. durch die sich hierbei ergebende Verlängerung der Verzögerung zwischen Auftreten und Detektion von Okklusionen vereitelt. Das vorgeschlagene Verfahren ermöglicht auch bei höheren Insulinkonzentrationen eine akzeptable Detektionsgeschwindigkeit, so dass kleinere und diskretere Pumpen verwendet werden können.

Die Okklusionserkennung tragbarer Infusionspumpen geschieht bei derzeitigen Systemen mittels einer Messung des Motorstroms und/oder der Reaktionskraft im Getriebe mittels Kraftsensor, wie z. B. in der US 2003/0073954, WO 0172357, US005647853 oder DE 19840992 beschrieben. Die Auswertung erfolgt im Wesentlichen durch Schwellwertvergleiche für die vom Antrieb auf den Ampullenstopfen ausgeübte Kraft F oder eine numerische Approximation ihrer zeitlichen Ableitung dF/dt z. B. durch Berechnung von Regressionsgeraden. Diese Verfahren besitzen eine lange Detektionsverzögerung, da die Messungen eine Okklusion nur langsam wiederspiegeln und von einer ganzen Reihe von Einflussgrößen abhängen, die im Allgemeinen in einem großen Bereich schwanken können (z. B. Reibung von Getriebe und Ampullenstopfen), so dass die Schwellwerte zur Vermeidung von Fehldetektionen hoch gewählt werden müssen. Eine signifikante Reduktion der Detektionsverzögerung würde mit den bekannten Verfahren daher eine Kontrolle aller Einflussgrößen in sehr engen Grenzen erfordern und wäre daher technisch aufwändig und teuer.

Das vorgeschlagene Verfahren zur schnellen Okklusionserkennung beruht auf der Echtzeit-Auswertung eines Messsignals, welches eine Okklusion z. B. durch eine (nahezu) schlagartige Änderung reflektiert. Der fluidische Druck ist als Messsignal besonders geeignet, da er gegenüber anderen Messsignalen das Auftreten einer Okklusion deutlich schneller und empfindlicher wiederspiegelt. Die Messung des Drucks erfolgt bevorzugt am Pumpenauslass vorzugsweise mit einem kommerziell erhältlichen Drucksensor (z. B. piezoresistive Wheatson'sche Brücke).

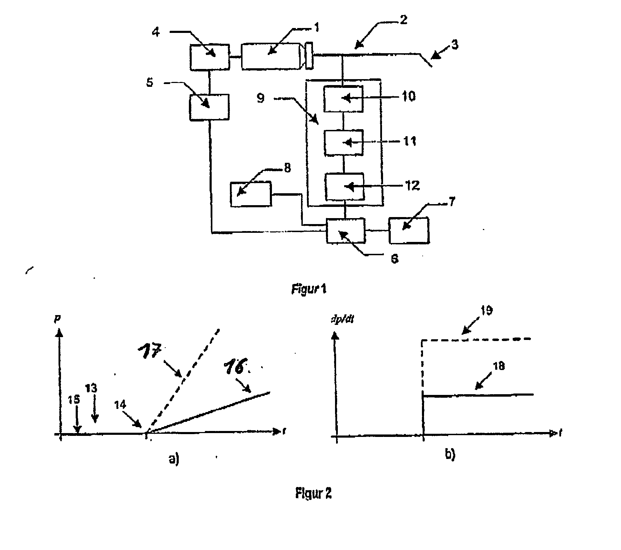

Die Figur 1 zeigt die hierfür relevanten Komponenten eines solchen Systems: Medikamentenreservoir 1, Katheter 2, Kanüle 3, Getriebe 4, Motor 5, Pumpensteuerung 6, Benutzerschnittstellen, wie z. B. Display, Buzzer, Vibrator, Tastatur 7, Energieversorgung 8 und Druckmessvorrichtung 9. Die Aufbereitung des vom Sensor 10 z. B. in Form einer Spannung bereitgestellten Messsignals erfolgt durch einen Verstärker 11 mit nachgeschaltetem Analog/Digital-Wandler 12.

Der Sensor 10 kann z. B. den Fluiddruck in der Ampulle 1, dem Katheter 2 und/oder der Kanüle 3 messen.

Für das Verfahren zur Okklusionserkennung ist eine kontinuierliche Erfassung des Drucksignals vorteilhaft, aber nicht erforderlich. Statt dessen können z. B. Einzelmessungen zu definierten Zeitpunkten durchgeführt werden, z. B. jeweils unmittelbar vor oder nach einer Medikamentenausschüttung oder in kontinuierlichen Abständen. Die genaue Wahl der Messzeitpunkte ist für die Okklusionserkennung nicht bedeutend. Um den Energieverbrauch der Messvorrichtung 9 möglichst gering zu halten, kann diese z. B. nicht kontinuierlich betrieben, sondern von der Pumpensteuerung 6 z. B. nur zu den Messzeitpunkten mit Energie aus der Versorgung 8 versorgt werden.

Nachfolgend erfolgt die Beschreibung der Signalauswertung für den Fall einer Abtastung mit konstantem Abtastintervall $\Delta t$. Im Falle eines variablen Abtastintervalls kann das selbe Verfahren genutzt werden. In diesem Fall können lediglich die Druckdifferenzen $p_i - p_{i-1}$, (mit 1 als fortlaufendem Messindex) und die zugehörigen

Differenzquotienten $\dfrac{p_i - p_{i-1}}{t_i - t_{i-1}}$ verwendet werden.

Die Okklusionserkennung erfolgt vorzugsweise durch einen Algorithmus auf dem Mikroprozessor der Pumpensteuerung 6. Eine Realisierung mit spezialisierten Hardwarekomponenten ist jedoch ebenfalls möglich.

Die Figur 2a zeigt schematisch den fluidischen Druck am Pumpenausgang 13, wenn zum Zeitpunkt $t_{Okklusion}$ 14 eine $O_{kklusion}$ auftritt. Figur 2b stellt die zeitliche Ableitung des Drucks dar. Vor Auftreten der Okklusion ist der Druck (bezogen auf den Umgebungsdruck) im Mittel null (15), anschließend steigt er annähernd linear an, wobei der Anstieg pro Zeiteinheit bei einer kleinen Förderrate geringer ist (16) als bei großer Förderrate (17), wie anhand der jeweiligen zeitlichen Ableitung (18, 19) erkennbar.

In der Praxis ist das Drucksignal jedoch mit einer Vielzahl sowohl hoch- als auch niederfrequenter Störungen behaftet, die in der selben Größenordnung wie der Druckanstieg liegen können, bei dem im Okklusionsfalle eine Alarmierung erfolgen soll. Es ist insbesondere ein typisches Charakteristikum beim permanenten Einsatz tragbarer Infusionspumpen, dass die geodätischen Höhen $h_{Pumpe}$ der Pumpe und des Infasionsortes (Sitz der Kanüle) $h_{Infusion}$ nicht identisch und auch nicht konstant sind (z. B. bei Bedienung der Pumpe, Wechsel des Infusionsortes, Hinlegen. etc.). Die Höhendifferenz $\Delta h= h_{Pumpe} - h_{Inifusion}$ bewirkt wegen des hydrostatischen Drucks der Medikamentensäule im Katheter ein hierzu proportionales Drucksignal.

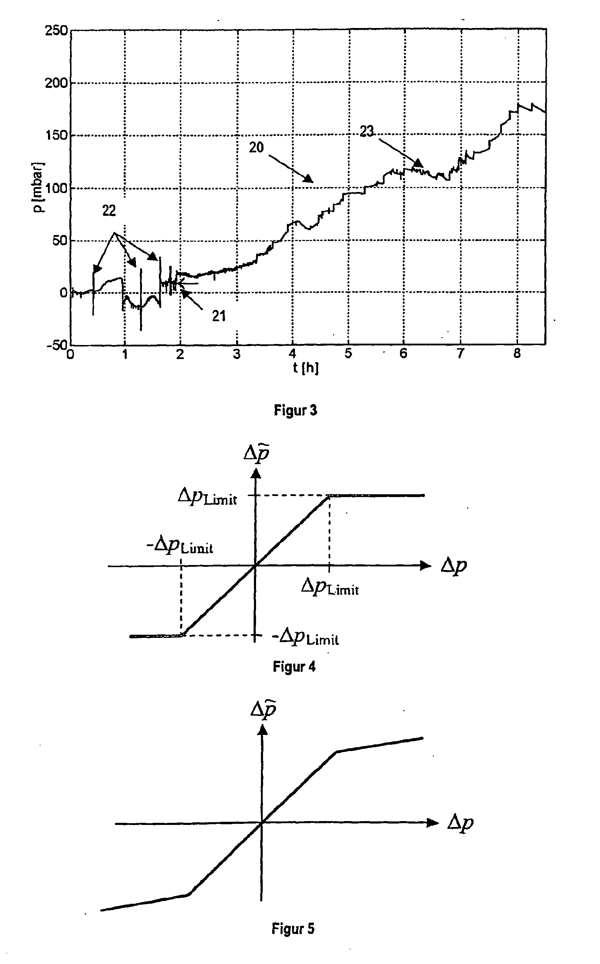

Die Figur 3 zeigt exemplarisch einen sich unter realen Bedingungen ergebenden Druckverlauf (20) bei einer Förderrate von $1\mu l$/h mit Okklusionszeitpunkt (21). Sowohl vor Beginn der Okklusion existieren deutliche Fluktuationen mit Druckspitzen (22) und auch nach Beginn der Okklusion gibt es längere Phasen konstanten oder fallenden Drucks (23).

Daher wird in dieser Erfindung vorgeschlagen, nicht das unmittelbare Drucksignal $p$ auszuwerten, sondern mit Hilfe einer geeigneten (nichtlinearen) Filterung ein modifiziertes Drucksignal $\tilde{p} = f(p)$ zu ermitteln, das folgenda Merkmale aufweist:

- Der im Falle einer Okklusion auftretende kontinuierliche Druckanstieg wird weitgehend verzögerungs- und abschwächungsfrei von $p$ nach $\tilde{p}$ übertragen.
- Sonstige Schwankungen und Sprünge werden so stark gedämpft, dass sie nicht zu einem Fehlalarm führen.

Einfache (lineare) Tiefpassfilter sind für diesen Zweck nicht geeignet, da hier der Druckanstieg einer Okklusion nur sehr langsam übertragen würde.

Die vorgeschlagene Filterung besteht aus einer Limitierung des Druckanstiegs $\Delta p = P_i - P_{i-1}$ zwischen den Abtastungen, wobei die Berechnung des modifizierten Drucksignals nach folgender Vorschrift erfolgt

$$\tilde{p}_0 = 0 \quad \text{(Initialisierung)}$$

$$\Delta \tilde{p}_i = f(\Delta p_i)$$

$$\tilde{p}_i = \tilde{p}_{i-1} + \Delta \tilde{p}_i$$

Dabei ist $f$ die den Druckanstieg limiterende Übertragungsfunktion. Im einfachsten Fall ist $f$ eine ungerade Funktion gemäs Figur 4, die aus drei linearen Segmenten mit den Steigungen null und eins besteht. Der Schwellwert $\Delta p_{Limit}$ legt die maximale Änderung des modifizierten Drucksignals pro Abtastintervall fest. Dieser ist so zu wählen, dass der im Fall einer Okklusion bei jeder Förderung auftretende Drucksprung $\Delta_{pOkklusion}$ möglichst voll übertragen wird, andererseits ein plötzliches Anheben oder Absenken der Pumpe kein Ansprechen des nachfolgenden Detektionsalgorithmus bewirkt. Wird zur Okklusionserkennung etwa ein einfacher Schwellwert $\lambda$ auf das modifizierte Drucksignal angewendet, so muss demnach gelten.

$$\Delta p_{Limit} \geq \Delta p_{Okklusion}$$
$$c \, \Delta p_{Limit} < \lambda$$

Dabei ist c eine von der geforderten Detektionssicherheit sowie der zulässigen Rate von Fehlalarmen bestimmte Konstante. Da $\Delta p_{Okklusion}$ mit zunehmender Förderrate ansteigt, soltte $\Delta p_{Limit}$ auch mit zunehmender Förderrate wachsend gewählt werden.

Neben der in Figur 4 dargestellten Funktion kann $f$ auch eine andere stückweise lineare Funktion mit

$$\frac{\Delta \tilde{p}}{\Delta p} = 1 \quad \text{für} \quad |\Delta p| \leq \Delta p_{Limit}$$

$$0 < \frac{\Delta \tilde{p}}{\Delta p} < 1 \quad \text{für} \quad |\Delta p| > \Delta p_{Limit}$$

gemäss Figur 5 sein, wodurch die Wahl von $\Delta p_{Okklusion}$ weniger kritisch wird. Alternativ können stetig diffierenzierbare Funktionen mit ähnlichem Verlauf verwendet werden.

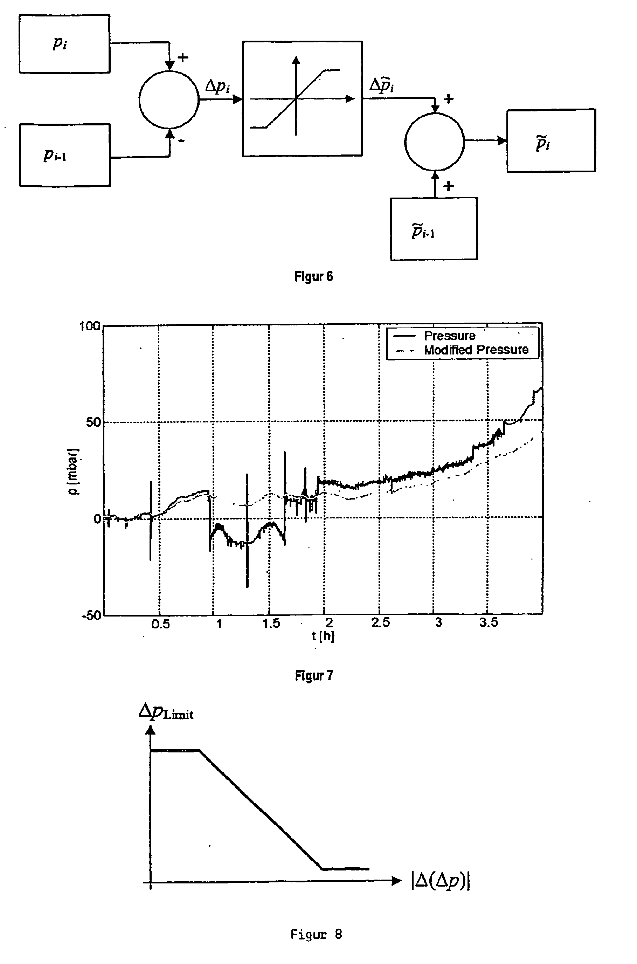

Figur 6 stellt den Signalfluss zur Gewinnung des modifizierten Drucksignals dar. Die Implementierung von f erfolgt durch analytische Funktionen oder durch eine Look-Up-Tafel.

In der oben gegebenen Formulierung wird

$\Delta p_{\text{Limit}}$ und damit die Übertragungsfunktion $f$ ausschliesslich auf Grundlage der aktuellen Förderrate festgelegt. Zur Erhöhung der Detektionssicherheit ist es jedoch sinnvoil, $f$ durch eine Enbeziehung des Rausch- und Störniveaus adaptiv zu gestalten. Dadurch lässt sich gewährleisten, dass einerseits bei geringem Störniveau (während ruhiger Tätigkeiten, Nachts, etc.) Okklusionen schnellstmöglich erkannt werden, ohne dass es bei hohem Störniveau (z. B. bei sportlicher Aktivität)) zu Fehlalarmen kommt. Als universelles und einfaches Mass für das Störniveau ist die zweite zweite Differenz $\Delta(\Delta p)$ mit

$$\Delta(\Delta p)_i = \Delta p_i - \Delta p_{i-1} = p_i - 2p_{i-1} + p_{i-2}.$$

geeignet, die eine Näherung der zweiten zeidichen Ableitung darstellen. Je kleine $\Delta(\Delta p)$ ist, umso grösser darf $\Delta p_{\text{Limit}}$ gewählt werden.[1] Die Wahl von $\Delta p_{\text{Limit}} = g(\Delta(\Delta p))$ erfolgt gemäss Figur 8 oder einer ähnlichen Funktion. Ferner ist es möglich, $\Delta p_{\text{Limit}}$ nicht aus einem einzelnen Wert von $\Delta(\Delta p)$ zu bestimmen, sondern für $\Delta(\Delta p)$ zunächst eine Glättung vorzusehen. Dies kann durch einen gleitenden Mittelwert oder andere lineare oder nichtlineare Tiefpassfilter geschehen.

Im auf diese Weise modifizierten Drucksignal $\tilde{p}$ ist eine Okklusionserkennung sehr einfach z. B. durch einen Schwellwart $\lambda$ möglich, bei dessen Überschreiten ein Okkuslionsalam ausgelöst wird. $\lambda$ kann dabei fest oder ebenfalls in Abhängigkeit der Förderrate gewählt werden.

Als besonders vorteilhaft erweist sich jedoch statt eines direkten Schweitwertes für $\tilde{p}$ die rekursive Berechnung einer Funktion $U$ (mit Intialisierung $U_1 - 0$) gemäss folgender Vorschrift:

$$U_i = U_{i-1} + \left(\Delta \tilde{p}_i - k\right)$$

$$m_i = \min_{1 \leq k \leq i} U_k$$

$$R_i = U_i - m_i$$

Dabei gibt $k$ den (von der Förderrate abhängigen) minimalen Druckanstieg pro Abtastung im Falle einer Okklusion an. Ein Druckanstieg mit Druckdifferenzen, die im Mittel unter unterhalb von $2k$ (z. B. durch Sensordrift) liegen, führt mit Sicherheit nicht zu einem Okklusionsalarm. Die Okklusionserkennung erfolgt durch Vergleich von $R$ mit einem Schwellwert $\lambda$.

$$\begin{cases} \text{wenn } R_i \leq \lambda : & \text{keine Okklusion} \\ \text{wenn } R_i > \lambda : & \text{Okklusion} \end{cases}$$

Die besondere Robustheit dieses Ansatzes lässt sich aus folgender Überlegung ersehen. Solange keine Okklusion vortlegt, sind die Terme $\Delta \tilde{p}_i - k$ im zeitlichen Mittel negativ, so dass auch die U, immer negativer werden (auch wenn es aufgrund von Rauschen auch positive Summanden gibt). Entsprechend wird auch min U immer negativer und unterscheidet sich nicht signifikant von U. (Ohne Ausreisser nach oben gälte zu jedem Zeitpunkt $m_i = U_i$). Daher ist $R$ immer positiv und ungefähr null. Einzelne "Ausreisser nach oben" von $\Delta \tilde{p}$ fuhren nicht zu einer signifikanten Erhöhung von U und damit auch nicht zur einer irrtümlichen Okklusionsdetektion.

Nach Auftreten einer Okklusion sind die Terme $\Delta \tilde{p}_i - k$ im zeitlichen Mittel positiv, so dass auch $U$ im Mittel ansteigt (auch wenn es aufgrund von Rauschen auch negative Summanden gibt). Da minU sich nicht weiter verändert, steigt $R$ nun eberifalls an, wobei die Steigung (aufgrund von $k$) etwas geringer ist als die von $\tilde{p}$. Einzelne "Ausreisser nach unten" von $\Delta \tilde{p}$ führen Insgesamt nicht zu einem signifikanten Abbau von $U$.

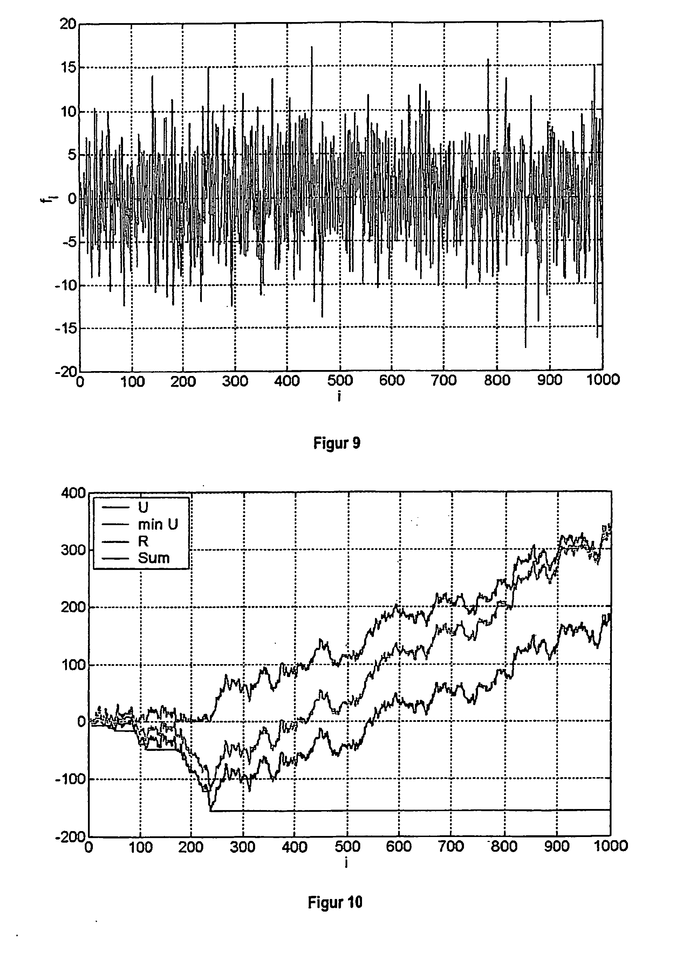

In Figur 9 sowie Figur 10 wird das Verhalten in einem Beispiel dargestellt. Figur 9 zeigt zunächst eine allgemeine Funktion $f$ dar (die den $\Delta \tilde{p}$ der Okbieonserkennung entspricht). Zunächst ist der Mittelwert $\bar{f}_1 = 0$, das überlagerte normalverteilte Rauschen hat eine Standardabweichung von $\sigma = 5$. Beim Index $\bar{i}_{\text{lump}} = 300$ springt der Mittelwert auf $\bar{f}_2 = 0.5$ (Beginn einer Okklusion), während das Rauschen unverändert bleibt. Figur 10 zeigt den Verlauf von $U$ (blau) $m = \min U$ (grün) sowie $R$ (rot). Zusätzlich ist zum Vergleich die Summe über die $f_i$, (türkis, entspricht $\tilde{p}$) dargestellt. Es ist ersichtlich, dass U zunächst im Mittel abnimmt und sein Minimum bei $i \approx 250$ erreicht (ohne Rauschen läge es bei $i = 300$), $R$ ist zunächst sehr klein. Für $i > i_{\text{Jump}}$ steigt U und damit auch $R$ kontinuierlich an. Für $i = 400$ liegt $R$ bereits deutHch über den grössten Werten vor dem Spring, während dies für die Summe der $f_i$ noch nicht gill, so dass $R$ für eine schnelle Detektion günstiger ist Mit Zunahme der Zeit nähem sich beide Funktionen immer weiter an.[2] Dieses Verfahren ist aus der Literatur als PAGE-HINKLEY-Stopping-Rule bekannt und es wurde nachgewiesen, dass es unter Annahme normalverteilten Rauschens die mittlere Detektionszeit minimiert.

Um auch unter sehr ungünstigen Bedingungen, in denen das Drucksignal aufgrund extremer Störungen nicht auswertbar ist, eine Okklusionsdetektion zu gestatten, ist die Kopplung des hier vorgeschlagenen Ansatzes

[1] Eine Bestimmung des Störniveaus basierend auf der einfachen Differenz $\Delta p$ ist nicht sinnvotl, da ansonsten der (annähernd) konstant Drukanstieg pro Abtastung bei einer Okklusion nicht voll übertragen wurde.

[2] Aufgrund von k würde die Summe der $f_i$ irgendwann auch grosser als $R$. mit einem oder mehreren der bisher genutzten Verfahren (Messung von Motorstrométriebereaktionskraft) möglich. Hierbei sind zwei Vorgehensweisen denkbar.

• Die verschiedenen Verfahren zur Okklusionserkennung arbeiten parallel und unabhängig von einander. Eine Okklusion wird immer dann angenommen, wenn sie nach einem der Verfahren detektiert wurde (ODER-Verknüpfung).

• Das Störniveau des Drucksignals wird permanent überwacht (z. B. durch Auswertung der zweiten zeitlichen Differenzen, siehe oben). Überschreitet das Störniveau einen Schwellwert oberhalb dessen eine sichere Auswertung nicht mehr gewährleistet ist, wird die druckbasierte Okklusionsmessung zur Vermeidung von Fehlalarmen vorübergehend deaktiviert und es wird statt dessen ein anderes Verfahren eingesetzt, wobei das Störniveau des Drucksignals weiterhin gemessen wird. Sinkt es unter einen gegeben Schwellwert, wird wieder auf die empfindlichere druckbasierte Okklusionserkennung gewechselt.

Eine besonders vorteilhafte Variante ergibt sich aus einer Koppelung beider Alternativen. Hierbei arbeiten die unterschiedlichen Verfahren parallel, wobei die Ausgangssignale der einzelnen Verfahren (z. B. *R* und *F* im Falle einer Messung von Druck- und Getriebereaktionskraft) mit Hilfe unscharfen (fuzzy) UND-Verknüpfung kombiniert werden. Dabei wird die Druckmessung im Vergleich zur Kraftmessung um so stärker gewichtet, je geringer das Rauschsignal ist Als Grenzwerte sehr geringer bzw. sehr grosser Störungen des Drucksignals ergeben sich die beiden oben genannten Alternativen.

Weiterhin ist es möglich, aus dem Drucksignal weitere Kriterien einer Okklusion zu generieren (z B. Steigung einer linearen oder nichtlinearen Regressionsfunktion) und sie mit dem hier vorgeschlagenen Verfahren durch eine Fuzzy-UND-Verknupfung zu kombinieren.

**Patentansprüche**

1. Verfahren zur Erkennung einer Okklusion, wobei ein Signal, welches einen Förderzustand eines Fluids charakterisieren kann, gemessen wird, und wobei das gemessene Signal gefiltert wird, **dadurch gekennzeichnet, dass** das gemessene Signal ein fluidischer Druck in einer Ampulle (1), einem Katheter (2) und/oder einer Kanüle (3) ist und dass mittels der Filterung ein modifiziertes Drucksignal ermittelt wird, wobei die Filterung aus einer Limitierung des Druckanstiegs zwischen zwei chronologisch aufeinanderfolgenden gemessenen Signalen mittels einer nicht linearen limitierenden Übertragungsfunktion besteht, und wobei eine Okklusion anhand des modifizierten Drucksignals mittels eines Schwellwerts erkannt wird.

2. Verfahren nach Anspruch 1, wobei die Messung des Signals kontinuierlich und/oder diskret vorgenommen wird.

3. Verfahren nach einem der vorhergehenden Ansprüche, wobei zur Filterung und/oder Auswertung des gemessenen Signals die jeweilige Förderrate berücksichtigt wird.

4. Verfahren nach einem der vorhergehenden Ansprüche, wobei zur Filterung und/oder Auswertung des gemessenen Signals das Störniveau des Drucksignals berücksichtigt wird.

5. Verfahren nach einem der vorhergehenden Ansprüche, wobei eine Okklusion durch die Verwendung der Page-Hinkley-Stopping-Rule detektiert wird.

6. Vorrichtung zum Detektieren einer Okklusion mit einem Sensor (10) zur Messung eines Fluiddrucks und einer Auswerteeinheit (9-12), **dadurch gekennzeichnet, dass** die Auswerteeinheit zur Durchführung eines Verfahrens nach einem der vorhergehenden Ansprüche ausgestaltet ist.

**Claims**

1. A method for detecting an occlusion, wherein a signal which can characterize a flow state of a fluid is measured and wherein the measured signal is filtered, **characterized in that** the measured signal is a fluidic pressure in an ampoule (1), a catheter (2) and/or a cannula (3) and that a modified pressure signal is determined by means of the filtering, wherein the filtering consists of a limiting of the pressure rise between two chronologically successive measured signals by means of a non-linear limiting transfer function, and wherein an occlusion is detected based on the modified pressure signal by means of a threshold value.

2. The method as claimed in claim 1, wherein the signal is measured continuously and/or discretely.

3. The method as claimed in one of the preceding claims, wherein the respective flow rate is taken into account in order to filter and/or evaluate the measured signal.

4. The method as claimed in one of the preceding claims, wherein the distortion level of the pressure signal is taken into account in order to filter and/or evaluate the measured signal.

5. The method as claimed in one of the preceding claims, wherein an occlusion is detected by the use of the Page-Hinckley stopping rule.

6. A device for detecting an occlusion having a sensor (10) for measuring a fluid pressure and an evaluation unit (9-12), **characterized in that** the evaluation unit is designed to carry out a method as claimed in one of the preceding claims.

## Revendications

1. Procédé de détection d'une occlusion, dans lequel un signal qui peut caractériser une condition de transport d'un fluide est mesuré et dans lequel le signal mesuré est filtré ;
caractérisé par le fait que le signal mesuré est une pression fluidique dans une ampoule (1), dans un cathéter (2) et/ou dans une canule (3) et **par le fait qu'**au moyen du filtrage, on détermine un signal de pression modifié, le filtrage étant constitué d'une limitation de la montée en pression entre deux signaux mesurés se succédant chronologiquement, à l'aide d'une fonction de transfert limitatrice non linéaire ; et
dans lequel une occlusion est détectée à l'aide d'une valeur de seuil sur le signal de pression modifié.

2. Procédé selon la revendication 1, dans lequel la mesure du signal s'opère de manière continue et/ou discrète.

3. Procédé selon l'une des revendications précédentes, dans lequel pour le filtrage et/ou l'analyse du signal mesuré, on prend en compte les différents débits de transport.

4. Procédé selon l'une des revendications précédentes, dans lequel pour le filtrage et/ou l'analyse du signal mesuré, on prend en compte le niveau de perturbation du signal de pression.

5. Procédé selon l'une des revendications précédentes, dans lequel une occlusion est détectée par l'utilisation de la règle d'arrêt de Page-Hinkley.

6. Dispositif de détection d'une occlusion comportant un capteur (10) de mesure de la pression d'un fluide et une unité d'analyse (9 à 12), **caractérisé par le fait que** l'unité d'analyse est conçue pour mettre en oeuvre un procédé selon l'une des revendications précédentes.

Figur 1

Figur 2

Figur 3

Figur 4

Figur 5

8

Figur 6

Figur 7

Figur 8

Figur 9

Figur 10

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- US 20030073954 A
- WO 0172357 A
- US 005647853 A
- DE 19840992